Europäisches Patentamt

(19) European Patent Office

Office européen des brevets

(11) Publication number: **0 068 706**
**B1**

(12) **EUROPEAN PATENT SPECIFICATION**

(45) Date of publication of patent specification: 05.12.84

(21) Application number: **82303089.5**

(22) Date of filing: **15.06.82**

(51) Int. Cl.³: **C 12 P 19/06**

(54) **Precipitation of xanthan gum.**

(30) Priority: **25.06.81 GB 8119707**

(43) Date of publication of application:
**05.01.83 Bulletin 83/01**

(45) Publication of the grant of the patent:
**05.12.84 Bulletin 84/49**

(84) Designated Contracting States:
**DE FR GB NL**

(56) References cited:
**FR-A-1 526 105**
**US-A-3 232 929**
**US-A-3 338 792**
**US-A-3 355 447**
**US-A-3 382 229**

(73) Proprietor: **Kelco Biospecialties Limited**
**22 Henrietta Street**
**London WC2E 8NB (GB)**

(72) Inventor: **Smith, Ian Humphrey**
**58 Fitzroy Crescent**
**Woodley Reading Berks RG5 4EX (GB)**

(74) Representative: **Ablewhite, Alan James et al**
**MARKS & CLERK 57/60 Lincoln's Inn Fields**
**London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

The present invention relates to the recovery of xanthan gum by precipitation.

Xanthan gum is an anionic exocellular polysaccharide produced during fermentation of carbohydrates by *Xanthomonas campestris* and other bacterial of the genum *Xanthomonas*. The gum is manufactured on an industrial scale, and is used, for example, as a thickener in foods and as a viscosity control agent during secondary recovery of oil.

Fermentation of a xanthan-producing microorganism on a continuous or batch basis gives a viscous broth or beer containing the desired gum. Various techniques are available for recovering the xanthan gum from the broth, with precipitation being by far the commonest technique.

The methods for precipitating xanthan gum from aqueous solution include: precipitation using an organic solvent which is a nonsolvent for xanthan gum, for example by addition of acetone, methanol, or isopropanol; precipitation at alkaline pH of a calcium or other divalent metal salt of the polymer; precipitation at acid pH of an aluminium salt of the polymer; or precipitation as a quaternary ammonium salt of the polymer.

The most favoured method for the recovery of xanthan gum is precipitation using about 45 to 60% by weight of azeotropic isopropanol added to the broth. This then gives xanthan gum a salt of the cations already present in the broth. Thus, the typical xanthan gum product is predominantly a mixed salt with potassium and sodium.

In order to reduce the amount of isopropanol required to bring about precipitation, it has been proposed to add potassium or sodium chloride along with the isopropanol. However, this proposal does not seem to have been adopted, possibly since chloride ion is corrosive when used in conventional stainless steel equipment, and azeotropic isopropanol without added electrolyte has continued to be the precipitating agent of choice.

According to the present invention, there is now provided a process for recovery of xanthan gum, wherein a salt of a divalent cation and an organic solvent are added to xanthan gum solution to precipitate the gum.

In the present process, divalent cations are added in an amount that of itself is insufficient to cause precipitation of the gum at the prevailing pH, temperature and other conditions. Equally, the organic solvent, a nonsolvent for xanthan gum, is added in an amount that of itself is insufficient to cause precipitation of all the gum under the prevailing conditions. However, acting together, the divalent cations and organic solvent can cause precipitation of substantially all the gum as a manageable hard precipitate.

Thus, the present invention is based on the discovery that an effective precipitating agent for precipitating xanthan gum can comprise a sub-precipitant amount of divalent cations together with a sub-precipitant amount of organic solvent.

The preferred divalent cations are of an alkaline earth metal, especially calcium, though other divalent cations can be used, for example zinc. Xanthan gum broth typically contains 1 to 3% gum and has a pH of 6 to 8; a suitable sub-precipitant amount of divalent cations will usually then be from 0.005 to 1, preferably 0.01 to 0.1 g equivalents/litre (N). More generally, for any xanthan gum solution, a suitable amount of divalent cations can be found by experimentation, for instance as described in the Examples below.

The preferred organic solvent for the present process is isopropanol, i.e. propan-2-ol, often referred to as IPA. A suitable sub-precipitant amount of IPA is typically less than two volumes of IPA for each volume of solution, with from 0.8 to 1.8 volumes per volume being preferred. These ratios are quoted on the basis that the IPA is an industrial grade of aqueous IPA containing about 82 w/w% IPA. Other grades of IPA, including 100% IPA can be used, with due allowance being made in the volume per volume figures given.

The diluent organic solvent need not be IPA. For example, the present process can employ methanol or other alkanols, or acetone or other ketones. The solvents can now be employed in sub-precipitant amounts for the present purposes, with the solvent: broth ratio typically being 2:1 or less.

In practice the divalent cations will usually be added to the xanthan gum solution before the isopropanol. Preferably, the solution contains 1 to 3% of the gum, with the solution typically taking the form of a fermented broth. If desired, the solution may be subjected to a heat treatment before, during or after the steps constituting a recovery process embodying the present invention. Heat treatments in general improve the xanthan gum product, but heat treatment before or during the recovery process of the invention can offer the additional benefit of a further reduction in the amount of organic solvent which is required. Suitable heat treatments include, for example, holding the solution at 100 to 130°C for 1 to 15 minutes say at about 125°C for about 2 minutes.

The gum precipitated by the present process can be separated in a conventional manner e.g. by decantation, and further treated as desired e.g. to remove excess solvent from the gum and/or to improve the granularity of the gum. Techniques for these and other treatments are known *per se* and further details are unnecessary.

By adoption of the present process it is possible to achieve an efficient recovery of xanthan gum using less organic solvent than would be required without addition of the divalent cations to the solution. Furthermore, less organic solvent is employed than would be required with addition of sodium or potassium chloride or other source of monovalent cations.

The present invention is illustrated by the following non-limiting examples:

For the Examples the following experimental procedure was employed.

2.5 to 3% xanthan gum broths from laboratory fermentations using Medium A or B of Table 1 were used either before heat-treatment (NHT) or after heat treatment (HT) at 125°C for 2 minutes.

TABLE 1
Media composition

| Ion | Medium A (low phosphate) equivs/l | Medium B (low citrate) equivs/l |
|---|---|---|
| $H^+$ | $1.131 \times 10^{-2}$ | $5.710 \times 10^{-3}$ |
| $NH_4^+$ | $2.906 \times 10^{-2}$ | $2.906 \times 10^{-2}$ |
| $K^+$ | $5.601 \times 10^{-4}$ | $1.117 \times 10^{-2}$ |
| $Mg^{2+}$ | $3.481 \times 10^{-3}$ | $3.266 \times 10^{-3}$ |
| $Ca^{2+}$ | $7.006 \times 10^{-4}$ | $7.006 \times 10^{-4}$ |
| $OH^-$ | $4.181 \times 10^{-3}$ | $3.408 \times 10^{-3}$ |
| $SO_4^{2-}$ | $5.601 \times 10^{-4}$ | $5.582 \times 10^{-4}$ |
| $HPO_4^{2-}$ | $2.906 \times 10^{-2}$ | $4.023 \times 10^{-2}$ |
| $citrate^{3-}$ | $1.131 \times 10^{-2}$ | $5.710 \times 10^{-3}$ |

Increasing volumes of $\underline{1M}$ solutions of various salts, $(Ca(NO_3)_2, MgSO_4, Na_2SO_4, Al(NO_3)_3)$, were added and well mixed with a high-speed stirrer into a series of 50 g aliquots of broth in 250 ml beakers. Each broth sample was then titrated and well mixed with 99% w/w isopropanol from a 250 ml burette up to the point where a hard precipitate was formed.

Where indicated, the titration data were plotted in the form of graphs of the per cent by weight of IPA in the water versus the logarithm of the normality of the added cation in the broth before alcohol addition. The calculated 82% IPA:broth (v/v) ratios are included on a separate scale (where the broth volume does not include the volume of the added salt solution).

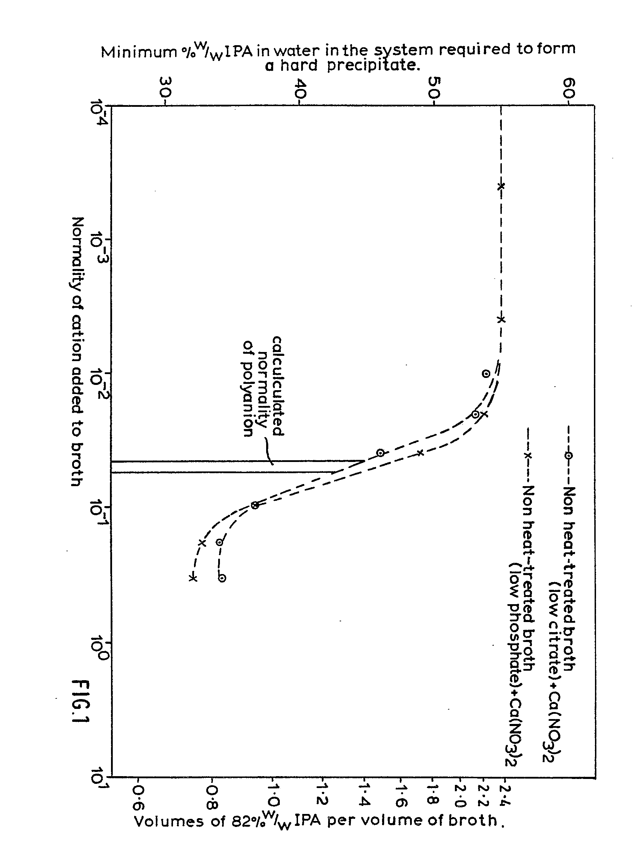

Examples 1 and 2
Figure 1 shows the curves for:

(E1) NHT broth (Medium A)$+Ca(NO_3)_2$

(E2) NHT broth (Medium B)$+Ca(NO_3)_2$

The results show that the quantity of IPA required to precipitate both NHT broths decreases very sharply over a concentration range of $Ca(NO_3)_2$ introduced to the broth extending from $2 \times 10^{-2}$ to $2 \times 10^{-1}$ $\underline{N}$ (i.e. 2.36 to 23.6 $gl^{-1}$ $Ca(NO_3)_2 . 4H_2O$ or 1.64 to 16.4 $gl^{-1}$ anhydrous $Ca(NO_3)_2$). To put this in perspective, the normality of the polyanion was calculated based on a polymer concentration of 25 to 30 $gl^{-1}$, a degree of pyruvylation x of 0.66, and an equivalent weight of the polyanion taken from the equation,

$$\text{E.W. of the polyanion} = \frac{865 + 69x}{1 + x}$$

The calculated normality falls in the range 4.5 to $5.5 \times 10^{-2}$ $\underline{N}$ which occurs in the centre of the linearly declining section of the graph.

The curves seen to plateau at high concentration of added $Ca(NO_3)_2$ (above $2 \times 10^{-1} \underline{N}$) indicating a constant minimum level of IPA to precipitate the gum irrespective of further salt addition.

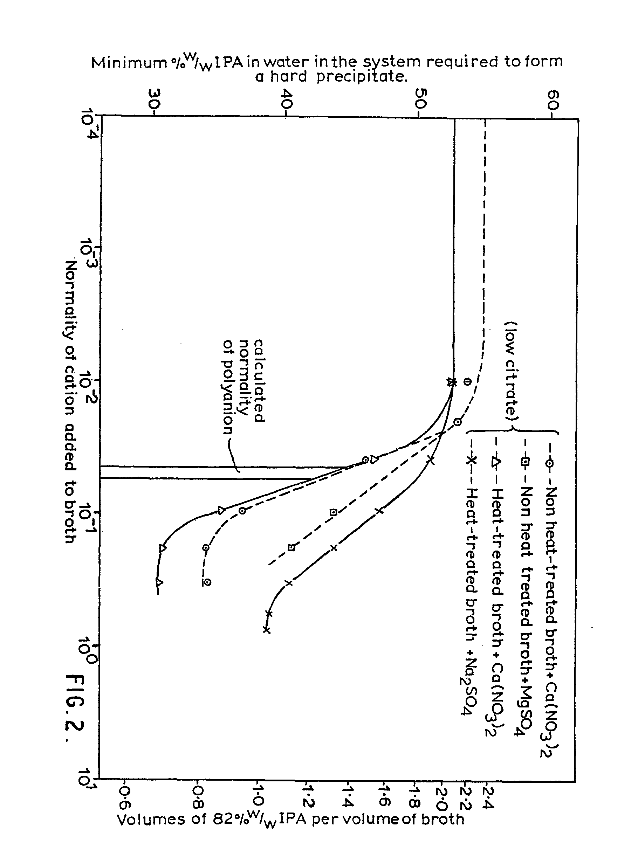

Examples 3 to 5 and Comparison Example 1
Figure 2 shows the curves for:

(E3) NHT broth (Medium A)$+Ca(NO_3)_2$

(E4) NHT broth (Medium A)$+MgSO_4$

(E5) HT broth (Medium A)$+Ca(NO_3)_2$

(CE1) HT broth (Medium A)$+Na_2SO_4$

where 'E' and 'CE' stand for 'Example' and 'Comparative Example' respectively.

The main features of interest from Fig. 2 are as follow:

(a) xanthan broth requires less IPA for precipitation after heat treatment

(b) Comparison of the two heat treated broths (E5) and (CE1) shows the exceptional improvement obtained using a divalent metal ion, calcium, in comparison with a monovalent metal ion, sodium. For example, at $0.3\underline{N}$, the calcium requires about 0.7 volumes of IPA, whereas the sodium requires about 1.05 volumes of IPA.

(c) $Ca(NO_3)_2$ is more effective in reducing the IPA requirement for precipitation than $Na_2SO_4$ or $MgSO_4$. This is not only so in terms of the reduction of IPA per unit concentration of added salt but also the maximum possible saving the IPA use is greater when $Ca(NO_3)_2$ is used. The latter observation may be due to the fact that $Na_2SO_4$ exceeds its solubility limit in IPA water mixtures before a sufficient concentration can be reached where the maximum IPA reduction is attained. The optimum level of $Ca(NO_3)_2$ addition for heat-

treated broth is $1.82 \times 10^{-1}$ $\underline{N}$ which is somewhere between 3.3 and 4 times the normality of the polyanion.

(d) The effect of $MgSO_4$ appears to be intermediate between that of $Ca(NO_3)_2$ and $Na_2SO_4$.

Example 6

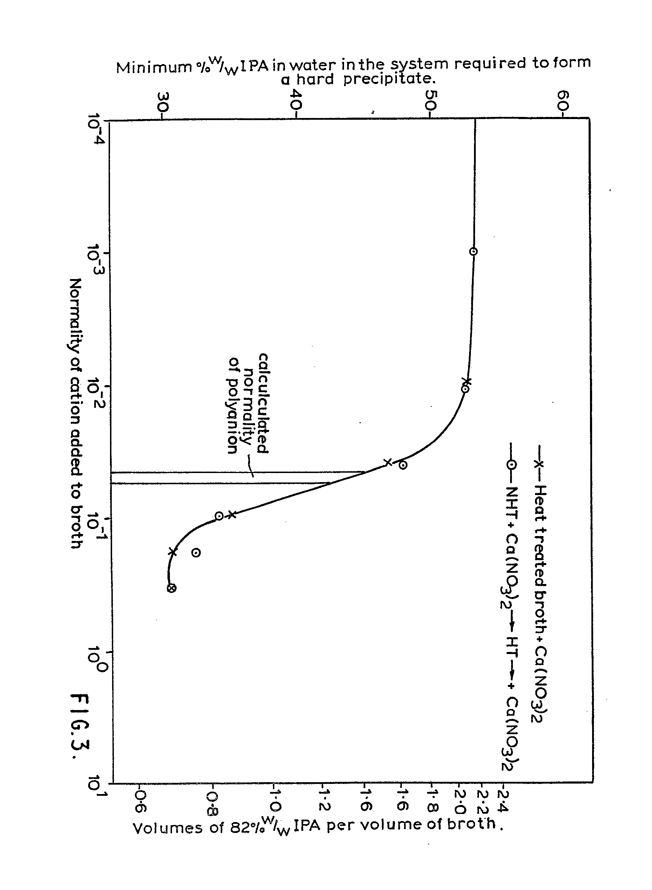

NHT broth (Medium A) was made $10^{-3}$ $\underline{N}$ in $Ca(NO_3)_2$ and then heat-treated. The IPA required to precipitate this broth as a function of the *total* added $Ca(NO_3)_2$ (before and after heat treatment) is shown in Fig. 3 in comparison with the corresponding data obtained from HT broth$+Ca(NO_3)_2$.

The results establish that the addition of a small quantity of $Ca(NO_3)_2$ prior to heat treatment of broth has no special effect in enhancing the ion-binding affinity of $Ca^{2+}$ ions. The IPA-added salt concentration profile remains essentially the same as that obtained for addition of all the salt after heat-treatment.

Comparison Example 2

$Al(NO_3)_3$ addition was investigated with NHT (Medium B) broth at 0.143 $\underline{N}$ $Al^{3+}$.

This created a very viscous and sticky mixture even before alcohol was introduced. When the alcohol concentration reached as little as 33.5% w/w in water clear rather glassy floccules were produced but these failed to harden into a manageable precipitate on addition of further IPA.

Examples 7 to 9 and Comparison examples 3 to 5

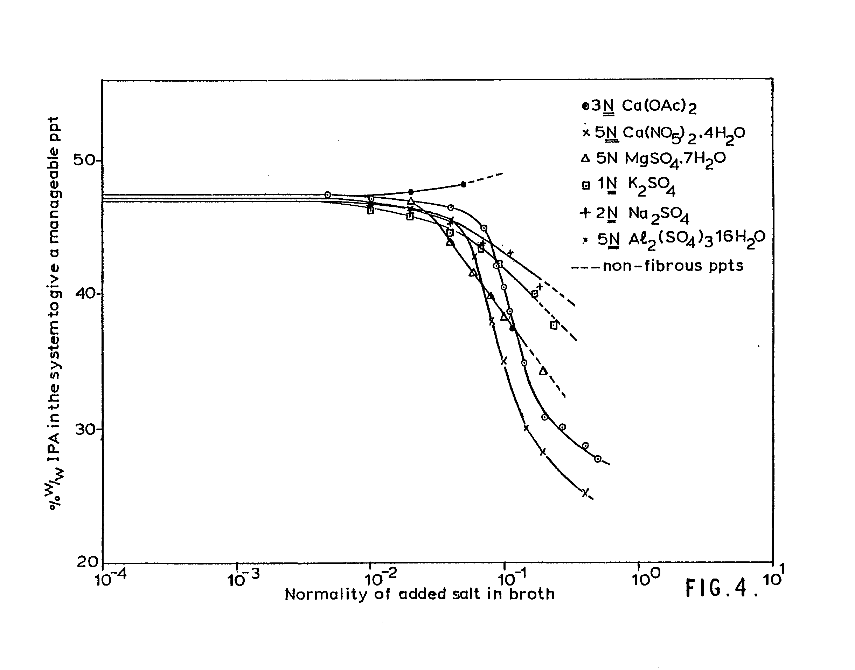

In a similar manner to the preceding figures, Figure 4 shows the curves for

(E7) HT broth$+3\underline{N}$ $Ca(OAc)_2$

(E8) HT broth$+5\underline{N}$ $Ca(NO_3)_2 . 4H_2O$

(E9) HT broth$+5\underline{N}$ $MgSO_4 . 7H_2O$

(CE3) HT broth$+1\underline{N}$ $K_2SO_4$

(CE4) HT broth$+2\underline{N}$ $Na_2SO_4$

(CE5) HT broth$+5\underline{N}$ $Al_2(SO_4)_3 . 16H_2O$

Among other features it will be seen that:

(a) The calcium salts give the best reduction in IPA, with nitrate being better than the acetate.

(b) The potassium and sodium salts are intermediate between the divalent and trivalent salts in efficacy.

(c) All but the calcium salts lead to non-fibrous precipitates.

(d) Magnesium gives the most reduction in IPA at lower concentrations before the curves fall away.

(e) The curves for $Ca(NO_3)_2$ and $Ca(OAc)_2$ both start dropping steeply at a relatively high concentration compared with other metal cations. Consequently, $MgSO_4$ is a good choice if only a small reduction in IPA is required.

**Claims**

1. In a process for precipitating xanthan gum from aqueous solution, the improvement which comprises adding to said solution an effective amount of precipitating agent which comprises a subprecipitant amount of divalent cations and a subprecipitant amount of an organic solvent.

2. The process of Claim 1, wherein the divalent cations are of zinc.

3. The process of Claim 1, wherein the aqueous solution is xanthan gum beer, the divalent cations are of an alkaline earth metal, and the organic solvent is an alkanol or ketone.

4. The process of Claim 3, wherein the solvent is isopropanol, methanol, or acetone.

5. The process of Claim 3, wherein the alkaline earth metal cations is of calcium or magnesium.

6. The process of Claim 3, wherein the beer contains 1 to 3% xanthan gum and has a pH of 6 to 8, the organic solvent is isopropanol, and the divalent cations are present in the amount 0.005 to 1 (preferably 0.01 to 0.1) g equivalents/litre.

7. The process of Claim 3, wherein the volume:volume ratio of solvent:beer is 0.8— 1.8:1 when the solvent is 82 w/w % IPA and less than 2:1 when the solvent is methanol or acetone.

8. The process of Claim 1 further comprising heat treating said solution.

9. The process of Claim 8, wherein the heat treatment occurs before or during the addition of said precipitation agent and comprises holding said solution at 100 to 130°C for 1— 15 minutes, preferably 125°C for 2 minutes.

**Patentansprüche**

1. Verfahren zur Ausfällung von Xanthangummi aus wässriger Lösung, dadurch verbessert, daß zu der Lösung eine wirksame Menge eines Ausfällungsmittels gefügt wird, das eine zur Auffällung nicht ausreichende Menge zweiwertiger Kationen und eine zur Ausfällung nicht ausreichende Menge eines organischen Lösungsmittels umfaßt.

2. Verfahren nach Anspruch 1 bei dem die zweiwertigen Kationen von Zink sind.

3. Verfahren nach Anspruch 1 bei dem die wässrige Lösung Xanthangummibier ist, die zweiwertigen Kationen von einem Erdallkalimetall sind und das organische Lösungsmittel ein Alkanol oder Keton ist.

4. Verfahren nach Anspruch 3 bei dem das Lösungsmittel Isopropanol, Methanol oder Aceton ist.

5. Verfahren nach Anspruch 3 bei dem die

Erdalkalimetallkationen von Calcium oder Magnesium sind.

6. Verfahren nach Anspruch 3 bei dem das Bier 1 bis 3% Xanthangummi enthält und einen pH von bis 8 hat, das organische Lösungsmittel Isopropanol ist und die zweiwertigen Kationen in der Menge von 0,005 bis 1 (vorzugsweise 0,01 bis 0,1) g-Äquivalente/Liter vorhanden sind.

7. Verfahren nach Anspruch 3, bei dem das volumen:Volumen-Verhältnis von Lösungsmittel:Bier 0,8—1,8:1 beträgt wenn das Lösungsmittel 82 Gew./Gew.-% IPA ist, und weniger als 2:1, wenn das Lösungsmittel Methanol oder Aceton ist.

8. Verfahren nach Anspruch 1 das darüber hinaus die Hitzebehandlung der Lösung umfaßt.

9. Verfahren nach Anspruch 8 bei dem die Hitzebehandlung vor oder während des Zusatzes des Ausfällungsmittels erfolgt und das Halten der Lösung bei 100 bis 130°C während 1—15 Minuten, vorzugsweise bei 125°C während 2 Minuten, umfaßt.

## Revendications

1. Dans un procédé de précipitation de gomme de xanthane à partir d'une solution aqueuse, le perfectionnement dans lequel on ajoute à ladite solutions une quantité efficace d'un agent précipitant comprenant une quantité subprécipitante de cations bivalents et une quantité subprécipitante d'un solvant organique.

2. Procédé de la revendication 1, où les cations bivalents sont de zinc.

3. Procédé de la revendication 1, où la solution aqueuse est une bière de gomme de xanthane, les cations bivalents sont d'un métal alcalino-terreux, et le solvant organique est un alcanol ou une cétone.

4. Procédé de la revendication 3, où le solvant est l'isopropanol, le méthanol ou l'acétone.

5. Procédé de la revendication 3, où le cation alcalino-terreux est de calcium ou de magnésium.

6. Procédé de la revendication 3, où la bière contient de 1 à 3% de gomme de xanthane et a un pH de 6 à 8, le solvant organique est l'isopropanol, et les cations bivalents sont présents en une quantité allant de 0,005 à 1 (dépréférence 0,01 à 0,1) g d'équivalents par litre.

7. Procédé de la revendication 3, où le rapport volume/volume du solvant:bière est de 0,8—1,8:1 lorsque le solvant est l'IPA à 82% p/p, et de moins de 2:1 lorsque le solvant est le méthanol ou l'acétone.

8. Procédé de la revendication 1 comprenant en outre le traitement thermique de ladite solution.

9. Procédé de la revendication 8, où le traitement thermique s'effectue avant ou pendant l'addition dudit agent de précipitation et implique de maintenir ladite solution entre 100 et 130°C pendant 1 à 15 minutes, de préférence 125°C pendant 2 minutes.

FIG.1

FIG. 2.

FIG.3.

FIG.4.

0 068 706